# EUROPÄISCHE PATENTANMELDUNG

(11) **EP 0 795 316 A1**
(43) Veröffentlichungstag der Anmeldung: **17.09.1997**
(21) Anmeldenummer: 97101878.3
(22) Anmeldetag: 06.02.1997
(51) Int. Cl.: A61K 7/48, A61K 7/50

(54) **Kosmetisch und/oder pharmazeutische Emulsionen enthaltend Partialglycerid(ether)sulfate, Emulgatoren und Chitosane**

(30) Priorität: 14.02.1996 DE 19605360
(71) Anmelder: Henkel Kommanditgesellschaft auf Aktien, 40589 Düsseldorf-Holthausen (DE)
(72) Erfinder: Fabry, Bernd, Dr. Dipl.-Chem., 41352 Korschenbroich (DE); Wachter, Rolf, Dr. Dipl.,Chem., 40595 Düsseldorf (DE); Behler, Ansgar, Dr. Dipl.-Chem., 46240 Bottrop (DE)

(57) **Zusammenfassung**

Es werden neue kosmetische und/oder pharmazeutische Emulsionen vorgeschlagen, enthaltend - bezogen auf den Emulgatoranteil - (a) 10 bis 50 Gew.-% Partialglycerid(ether)sulfate, (b) 50 bis 90 Gew.-% Emulgatoren und (c) 0,1 bis 10 Gew.-% Chitosane, mit der Maßgabe, daß sich die Mengenangaben zu 100 Gew.-% ergänzen. Die Emulsionen zeichnen sich auch bei Temperaturbelastung durch eine hohe Lagerstabilität aus und besitzen besonders vorteilhafte sensorische Eigenschaften.

## Beschreibung

### Gebiet der Erfindung

Die Erfindung betrifft kosmetische und pharmazeutische Emulsionen mit verbesserter Stabilität, enthaltend Partialglycerid(ether)sulfate, Emulgatoren und Chitosane in ausgewählten Mischungsverhältnissen.

### Stand der Technik

Aus der Europäischen Patentschrift **EP-B1 0 553 241** (SEPPIC) ist die Verwendung von Mischungen aus Alkyloligoglucosiden, Fettalkoholen und gegebenenfalls Polyglucose zur Herstellung von Emulsionen bekannt. Auch gemäß der Lehre der internationalen Patentanmeldung **WO 92/07543** (Henkel) lassen sich Alkyloligoglucoside zusammen mit Fettalkoholen und Partialglyceriden als kosmetische Emulgatoren einsetzen.

Zur Herstellung kosmetischer und pharmazeutischer Mittel, wie beispielsweise Cremes, Lotionen oder Salben, werden neben Ölkörpern in vielen Fällen auch Tenside eingesetzt. Es zeigt sich dabei, daß die resultierenden Emulsionen zwar bei Raumtemperatur stabil sind und eine ausreichend hohe Viskosität aufweisen, die jedoch bei längerer Lagerung, zumal bei Temperaturbelastung, allmählich zusammenbricht. Es entstehen dünnflüssige Produkte, in einer Reihe von Fällen kann es auch zu Entmischungen kommen. Außerdem sind auch die sensorischen Eigenschaften nicht immer zufriedenstellend.

Die Aufgabe der Erfindung hat somit darin bestanden, Emulsionen für kosmetische oder pharmazeutische Anwendungen zur Verfügung zu stellen, die frei von den geschilderten Nachteilen des Stands der Technik sind, d.h. auch bei Temperaturbelastung eine hohe Lagerstabilität aufweisen.

### Beschreibung der Erfindung

Gegenstand der Erfindung sind kosmetische und/oder pharmazeutische Emulsionen, enthaltend - bezogen auf den Emulgatoranteil -
(a) 10 bis 50 Gew.-% Partialglycerid(ether)sulfate
(b) 50 bis 90 Gew.-% Emulgatoren und
(c) 0,1 bis 10 Gew.-% Chitosane,
mit der Maßgabe, daß sich die Mengenangaben zu 100 Gew.-% ergänzen.

Überraschenderweise wurde gefunden, daß Mischungen von Partialglyceridethersulfaten, insbesondere Kokosmonoglyceridsulfaten, und Emulgatoren, vorzugsweise Alkyl- und/oder Alkenyloligoglucosiden, innerhalb definierter Einsatzverhältnisse zusammen mit Chitosanen Emulsionen ergeben, die lagerstabil sind, d.h. deren Viskosität sich auch bei längerer Lagerung bei erhöhten Temperaturen nicht verändert. Die Erfindung schließt die Erkenntnis ein, daß die Auswahl der Zusatzstoffe für die Stabilität der Emulsionen entscheidend ist, da mit anderen anionischen Tensiden bzw. anderen polymeren Filmbildnem nur Emulsionen einer wesentlich geringeren Stabilität erhalten werden. Die Erfindung schließt die Erkenntnis ein, daß die neuen Mittel zudem sensorisch deutlich besser beurteilt werden.

### Partialglycerid(ether)sulfate

Partialglycerid(ether)sulfate, bei denen es sich vorzugsweise um technische Monoglyceridsulfate und - ethersulfate handelt, stellen bekannte anionische Tenside dar, die nach den einschlägigen Methoden der präparativen organischen Chemie erhalten werden können. Üblicherweise geht man zu ihrer Herstellung von Triglyceriden aus, die gegebenenfalls nach Ethoxylierung zu den Monoglyceriden umgeestert und nachfolgend sulfatiert und neutralisiert werden. Gleichfalls ist es möglich, die Partialglyceride mit geeigneten Sulfatierungsmitteln, vorzugsweise gasförmiges Schwefeltrioxid oder Chlorsulfonsäure umzusetzen [vgl. **WO 92/09 569**, **WO 92/09 570** (Henkel)]. Die neutralisierten Stoffe können - falls gewünscht - einer Ultrafiltration unterworfen werden, um den Elektrolytgehalt auf ein gewünschtes Maß zu vermindern [**DE-A1 42 04 700** (Henkel)]. Übersichten zur Chemie der Monoglyceridsulfate sind beispielsweise von A.K.Biswas et al. in **J.Am.Oil.Chem.Soc. 37, 171 (1960)** und F.U.Ahmed in **J.Am.Oil.Chem.Soc. 67, 8 (1990)** erschienen. Aus der **WO 95/06702** (Henkel) sind Detergensgemische, enthaltend Monoglyceridsulfate und Alkyloligoglucoside bekannt. Mischungen von Monoglyceridsulfaten mit Proteinabbauprodukten bzw. acylierten Aminosäuren werden in der **DE-C1 44 33 071** (Henkel) beansprucht.

Die im Sinne der Erfindung einzusetzenden Partialglycerid(ether)sulfate folgen der Formel **(I)** 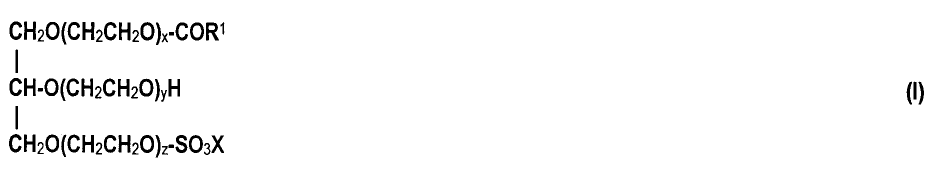 in der R¹CO für einen linearen oder verzweigten Acylrest mit 6 bis 22 Kohlenstoffatomen, x, y und z in Summe für 0 oder für Zahlen von 1 bis 30, vorzugsweise 2 bis 10, und X für ein Alkali- oder Erdalkalimetall steht. Typische Beispiele für im Sinne der Erfindung geeignete Partial- bzw. Monoglycerid(ether)sulfate sind die Umsetzungsprodukte von Laurinsäuremonoglycerid, Kokosfettsäuremonoglycerid, Palmitinsäuremonoglycerid, Stearinsäuremonoglycerid, Ölsäuremonoglycerid und Talgfettsäuremonoglycerid sowie deren Ethylenoxidaddukte mit Schwefeltrioxid oder Chlorsulfonsäure in Form ihrer Natriumsalze. Vorzugsweise werden Monoglyceridsulfate der Formel **(I)** eingesetzt, in der R¹CO für einen linearen Acylrest mit 8 bis 18 Kohlenstoffatomen steht.

### Emulgatoren

Als Emulgatoren können nichtionogene, ampholytische und/oder zwitterionische grenzflächenaktive Verbindungen verwendet werden, die sich durch eine lipophile, bevorzugt lineare Alkyl- oder Alkenylgruppe und mindestens eine hydrophile Gruppe auszeichnen. Diese hydrophile Gruppe kann sowohl eine ionogene als auch eine nichtionogene Gruppe sein. Nichtionogene Emulgatoren enthalten als hydrophile Gruppe z. B. eine Polyolgruppe, eine Polyalkylenglycolethergruppe oder eine Kombination aus Polyol- und Polyglycolethergruppe.

Bevorzugt sind solche Mittel, die als **O/W-Emulgatoren** nichtionogene Tenside aus mindestens einer der folgenden Gruppen enthalten:
(a1) Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe;
(a2) C_{12/18}-Fettsäuremono- und -diester von Anlagerungsprodukten von 1 bis 30 Mol Ethylenoxid an Glycerin;
(a3) Glycerinmono- und -diester und Sorbitanmono- und -diester von gesättigten und ungesättigten Fettsäuren mit 6 bis 22 Kohlenstoffatomen und deren Ethylenoxidanlagerungsprodukte;
(a4) Alkylmono- und -oligoglycoside mit 8 bis 22 Kohlenstoffatomen im Alkylrest und deren ethoxylierte Analoga;
(a5) Anlagerungsprodukte von 15 bis 60 Mol Ethylenoxid an Ricinusöl und/oder gehärtetes Ricinusöl;
(a6) Polyol- und insbesondere Polyglycerinester wie z.B. Polyglycerinpolyricinoleat oder Polyglycerinpoly-12-hydroxystearat. Ebenfalls geeignet sind Gemische von Verbindungen aus mehreren dieser Substanzklassen.

Die Anlagerungsprodukte von Ethylenoxid und/oder von Propylenoxid an Fettalkohole, Fettsäuren, Alkylphenole, Glycerinmono- und -diester sowie Sorbitanmono- und -diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind aus **DE-PS 20 24 051** als Rückfettungsmittel für kosmetische Zubereitungen bekannt.

C_{8/18}-Alkylmono- und oligoglycoside, ihre Herstellung und ihre Verwendung als oberflächenaktive Stoffe sind beispielsweise aus **US 3,839,318, US 3,707,535, US 3,547,828, DE-OS 19 43 689, DE-OS 20 36 472** und **DE-A1 30 01 064** sowie **EP-A 0 077 167** bekannt. Ihre Herstellung erfolgt insbesondere durch Umsetzung von Glucose oder Oligosacchariden mit primären Alkoholen mit 8 bis 18 C-Atomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl Betaine* bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C_{12/18}-Acylsarcosin.

Als **W/O-Emulgatoren** kommen in Betracht:
(b1) Anlagerungsprodukte von 2 bis 15 Mol Ethylenoxid an Ricinusöl und/oder gehärtetes Ricinusöl;
(b2) Partialester auf Basis linearer, verzweigter, ungesättigter bzw. gesättigter C_{12/22}-Fettsäuren, Ricinolsäure sowie 12-Hydroxystearinsäure und Glycerin, Polyglycerin, Pentaerythrit, Dipentaerythrit, Zuckeralkohole (z.B. Sorbit) sowie Polyglucoside (z.B. Cellulose);
(b3) Trialkylphosphate;
(b4) Wollwachsalkohole;
(b5) Polysiloxan-Polyalkyl-Polyether-Copolymere bzw. entsprechende Derivate;
(b6) Mischester aus Pentaerythrit, Fettsäuren, Citronensäure und Fettalkohol gemäß **DE-PS 11 65 574** sowie
(b7) Polyalkylenglycole.

Der Anteil der Emulgatoren am nicht-wäßrigen Anteil der Emulsionen kann 0,1 bis 10 und vorzugsweise 1 bis 7 Gew.-% betragen.

### Chitosane

Chitosane stellen Biopolymere dar und werden zur Gruppe der Hydrokolloide gezählt. Chemisch betrachtet handelt es sich um partiell deacetylierte Chitine unterschiedlichen Molekulargewichtes, die den folgenden - idealisierten - Monomerbaustein enthalten:

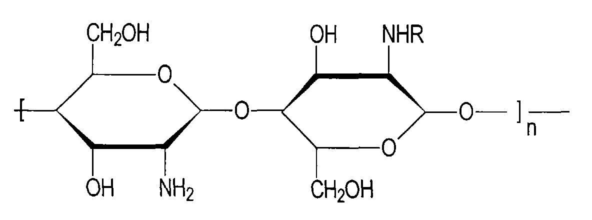

Im Gegensatz zu den meisten Hydrokolloiden, die im Bereich biologischer pH-Werte negativ geladen sind, stellen Chitosane unter diesen Bedingungen kationische Biopolymere dar. Die positiv geladenen Chitosane können mit entgegengesetzt geladenen Oberflächen in Wechselwirkung treten und werden daher in kosmetischen Haar- und Körperpflegemitteln sowie pharmazeutischen Zubereitungen eingesetzt (vgl. **Ullmann's Encyclopedia of Industrial Chemistry, 5th Ed., Vol. A6, Weinheim, Verlag Chemie, 1986, S. 231-332)**. Übersichten zu diesem Thema sind auch beispielsweise von B.Gesslein et al. in **HAPPI 27, 57 (1990)**, O.Skaugrud in **Drug Cosm.Ind. 148, 24 (1991)** und E.Onsoyen et al. in **Seifen-Öle-Fette-Wachse 117, 633 (1991)** erschienen.

Zur Herstellung der Chitosane geht man von Chitin, vorzugsweise den Schalenresten von Krustentieren aus, die als billige Rohstoffe in großen Mengen zur Verfügung stehen. Das Chitin wird dabei in einem Verfahren das erstmals von Hackmann et al. beschrieben worden ist, üblicherweise zunächst durch Zusatz von Basen deproteiniert, durch Zugabe von Mineralsäuren demineralisiert und schließlich durch Zugabe von starken Basen deacetyliert, wobei die Molekulargewichte über ein breites Spektrum verteilt sein können. Entsprechende Verfahren sind beispielsweise aus **Makromol.Chem. 177, 3589 (1976)** oder der französischen Patentanmeldung **FR-A 27 01 266** bekannt.

### Gewerbliche Anwendbarkeit

Die erfindungsgemäßen Emulsionen zeichnen sich auch bei Temperaturbelastung durch eine hohe Lagerstabilität sowie besonders vorteilhafte sensorische Eigenschaften aus. In einer bevorzugten Ausführungsform der Erfindung enthalten die Emulsionen - bezogen auf den Emulgatoranteil - 20 bis 40 Gew.-% Kokosmonoglyceridsulfat, 60 bis 80 Gew.-% Emulgatoren und 1 bis 5 Gew.-% Chitosane. Der nichtwäßrige Anteil der Emulsionen, der sich weitgehend aus dem Emulgator- und dem Ölkörpergehalt zusammensetzt und dabei in der Regel dem Feststoffgehalt entspricht, liegt üblicherweise bei 5 bis 95 und vorzugsweise 15 bis 75 Gew.-%. Das bedeutet umgekehrt, daß die Emulsionen 5 bis 95 und vorzugsweise 25 bis 85 Gew.-% Wasser enthalten können, abhängig davon, ob Lotionen mit einer vergleichsweise niedrigen oder Cremes und Salben mit einer hohen Viskosität hergestellt werden sollen.

### Ölkörper

Als **Ölkörper** kommen beispielsweise Guerbetalkohole auf Basis von Fettalkoholen mit 6 bis 18, vorzugsweise 8 bis 10 Kohlenstoffatomen, Ester von linearen C₆-C₂₀-Fettsäuren mit linearen C₆-C₂₀-Fettalkoholen, Ester von verzweigten C₆-C₁₃-Carbonsäuren mit linearen C₆-C₂₀-Fettalkoholen, Ester von linearen C₆-C₁₈-Fettsäuren mit verzweigten Alkoholen, insbesondere 2-Ethylhexanol, Ester von linearen und/oder verzweigten Fettsäuren mit mehrwertigen Alkoholen (wie z.B. Dimerdiol oder Trimerdiol) und/oder Guerbetalkoholen, Triglyceride auf Basis C₆-C₁₀-Fettsäuren, pflanzliche Öle, verzweigte primäre Alkohole, substituierte Cyclohexane, Guerbetcarbonate, Dialkylether und/oder aliphatische bzw. naphthenische Kohlenwasserstoffe in Betracht. Der Anteil der Ölkörper am nicht-wäßrigen Anteil der Emulsionen kann 5 bis 99 und vorzugsweise 10 bis 75 Gew.-% ausmachen

### Hilfs- und Zusatzstoffe

Die Emulsionen können als Hautpflegemittel, wie beispielsweise Tagescremes, Nachtcremes, Pflegecremes, Nährcreme, Bodylotions, Salben und dergleichen eingesetzt werden und als weitere Hilfs- und Zusatzstoffe Überfettungsmittel, Konsistenzgeber, Verdickungsmittel, Kationpolymere, Siliconverbindungen, biogene Wirkstoffe, Konservierungsmittel, Farb- und Duftstoffe enthalten.

Als **Überfettungsmittel** können Substanzen wie beispielsweise polyethoxylierte Lanolinderivate, Lecithinderivate, Polyolfettsäureester, Monoglyceride und Fettsäurealkanolamide verwendet werden, wobei die letzteren gleichzeitig als Schaumstabilisatoren dienen. Als **Konsistenzgeber** kommen in erster Linie Fettalkohole mit 12 bis 22 und vorzugsweise 16 bis 18 Kohlenstoffatomen in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten. Geeignete **Verdickungsmittel** sind beispielsweise Polysaccharide, insbesondere Xanthan-Gum, Guar-Guar, Agar-Agar, Alginate und Tylosen, Carboxymethylcellulose und Hydroxyethylcellulose, ferner höhermolekulare Polyethylenglycolmono- und -diester von Fettsäuren, Polyacrylate, Polyvinylalkohol und Polyvinylpyrrolidon, Tenside wie beispielsweise Fettalkoholethoxylate mit eingeengter Homologenverteilung oder Alkyloligoglucoside sowie Elektrolyte wie Kochsalz und Ammoniumchlorid.

Geeignete **kationische Polymere** sind beispielsweise kationischen Cellulosederivate, kationischen Stärke, Copolymere von Diallylammoniumsalzen und Acrylamiden, quaternierte Vinylpyrrolidon/Vinylimidazol-Polymere wie z.B. Luviquat® (BASF AG, Ludwigshafen/ FRG), Kondensationsprodukte von Polyglycolen und Aminen, quaternierte Kollagenpolypeptide wie beispielsweise Lauryldimonium hydroxypropyl hydrolyzed collagen (Lamequat®L, Grünau GmbH), quaternierte Weizenpolypeptide, Polyethylenimin, kationische Siliconpolymere wie z.B. Amidomethicone oder Dow Corning, Dow Corning Co./US, Copolymere der Adipinsäure und Dimethylaminohydroxypropyldiethylentrimamin (Cartaretine®, Sandoz/CH), Polyaminopolyamide wie z.B. beschrieben in der **FR-A 22 52 840** sowie deren vernetzte wasserlöslichen Polymere, kationische Chitinderivate wie beispielsweise quaterniertes Chitosan, gegebenenfalls mikrokristallin verteilt, kationischer Guar-Gum wie z.B. Jaguar® CBS, Jaguar® C-17, Jaguar® C-16 der Celanese/US, quaternierte Ammoniumsalz-Polymere wie z.B. Mirapol® A-15, Mirapol® AD-1, Mirapol® AZ-1 der Miranol/US.

Geeignete **Siliconverbindungen** sind beispielsweise Dimethylpolysiloxane, Methyl-phenylpolysiloxane, cyclische Silicone sowie amino-, fettsäure-, alkohol-, polyether-, epoxy-, fluor- und/oder alkylmodifizierte Siliconverbindungen. Unter **biogenen Wirkstoffen** sind beispielsweise Pflanzenextrakte und Vitaminkomplexe zu verstehen. Als **Konservierungsmittel** eignen sich beispielsweise Phenoxyethanol, Formaldehydlösung, Parabene, Pentandiol oder Sorbinsäure. Als **Perlglanzmittel** kommen beispielsweise Glycoldistearinsäureester wie Ethylenglycoldistearat, aber auch Fettsäuremonoglycolester in Betracht. Als **Farbstoffe** können die für kosmetische Zwecke geeigneten und zugelassenen Substanzen verwendet werden, wie sie beispielsweise in der Publikation **"Kosmetische Färbemittel" der Farbstoffkommission der Deutschen Forschungsgemeinschaft, Verlag Chemie, Weinheim, 1984, S.81-106** zusammengestellt sind. Diese Farbstoffe werden üblicherweise in Konzentrationen von 0,001 bis 0,1 Gew.-%, bezogen auf die gesamte Mischung, eingesetzt.

Der Gesamtanteil der Hilfs- und Zusatzstoffe kann 1 bis 10, vorzugsweise 2 bis 5 Gew.-% - bezogen auf die Mittel - betragen. Die Herstellung der Mittel kann in an sich bekannter Weise, d.h. beispielsweise durch Heiß-, Heiß-Heiß/Kalt- bzw. PIT-Emulgierung erfolgen. Hierbei handelt es sich um ein rein mechanisches Verfahren, eine chemische Reaktion findet nicht statt.

Typische Emulsionen weisen Zusammensetzungen gemäß Tabelle 1 auf (mit der Maßgabe, daß sich die Angaben zu 100 Gew.-% ergänzen).

**Tabelle 1**

| Typische Zusammensetzung von Lotionen und Cremes | | |
|---|---|---|
| **Bestandteil** | **Lotion [Gew.-%]** | **Creme [Gew.-%]** |
| Partialglycerid(ether)sulfate | 1 - 2 | 1 - 3 |
| Emulgatoren | 2 - 3 | 2 - 6 |
| Chitosane | 0,05 - 0,2 | 0,05 - 0,2 |
| Ölkörper | 25 - 50 | 25 - 50 |
| Hilfs- und Zusatzstoffe | 1 - 5 | 1 - 5 |
| Wasser | 50 - 70 | 25 bis 50 |

### Beispiele

Es wurden Emulsionen hergestellt, enthaltend 35 Gew.-% Ölkörper (D1 bis D3), 5 Gew.-% Tensid/Emulgator-Mischungen mit und ohne Zusatz von Chitosan und ad 100 Gew.-% Wasser. Die Herstellung der Emulsionen erfolgte nach der PIT-Methode, also oberhalb der Phaseninversionstemperatur. Die Rezepturen R1 und R2 enthalten die Kombination der Komponenten A1 bis A3 und sind erfindungsgemäß. Die Rezepturen R3 bis R5 enthalten zwar die erfindungsgemäße Tensid/Emulgator-Kombination A1/A2, jedoch andere polymere Filmbildner (B1 bis B3). Die Rezepturen R6 bis R8 enthalten schließlich zwar die erfindungsgemäße Emulgator/Chitosan-Kombination, jedoch andere Tenside (C1 bis C3). Die Viskosität der Proben wurde nach der Brookfield-Methode in einem RVF-Viskosimeter (20 Upm, Spindel 1) sofort sowie nach Lagerung über 7 Tage bei 20 bzw. 40°C bestimmt. Die Ergebnisse sind in Tabelle 2 zusammengefaßt.

### Eingesetzte Substanzen (CTFA-Nomenklatur, soweit möglich)

A1) Kokosmonoglyceridsulfat-Natriumsalz
A2) Kokosalkyloligoglucosid (Plantaren® APG 2000, Henkel KGaA)
A3) Kationisches Biopolymer vom Chitosan-Typ (Hydagen® CMF, Henkel KGaA)
B1) Polyvinylpyrrolidon
B2) Polyvinylpyrrolidon-Vinylacetat-Copolymere
B3) Hyaluronsäure
C1) Kokosfettalkohol+2EO-sulfat-Natriumsalz
C2) Kokosfettalkohol+3EO-sulfosuccinat-Di-Natriumsalz
C3) Kokosfettsäure+5EO-ethercarbonsäure-Natriumsalz
D1) Dicapryl Ether
D2) Decyl Oleate
D3) Mandelöl

**Tabelle 2**

| Viskosität und Lagerstabilität (Mengenangaben als Gew.-%) | | | | | | | | |
|---|---|---|---|---|---|---|---|---|
| **Komponenten** | **R1** | **R2** | **R3** | **R4** | **R5** | **R6** | **R7** | **R8** |
| A1 | 1,9 | 2,30 | 1,9 | 1,9 | 1,9 | - | - | - |
| A2 | 3,0 | 2,25 | 3,0 | 3,0 | 3,0 | 3,0 | 3,0 | 3,0 |
| A3 | 0,1 | 0,2 | - | - | - | 0,1 | 0,1 | 0,1 |
| B1 | - | - | 0,1 | - | - | - | - | - |
| B2 | - | - | - | 0,1 | - | - | - | - |
| B3 | - | - | - | - | 0,1 | - | - | - |
| C1 | - | - | - | - | - | 1,9 | - | - |
| C2 | - | - | - | - | - | - | 1,9 | - |
| C3 | - | - | - | - | - | - | - | 1,9 |
| D1 | 15 | 15 | 15 | 15 | 15 | 15 | 15 | 15 |
| D2 | 10 | 10 | 10 | 10 | 10 | 10 | 10 | 10 |
| D3 | 5 | 5 | 5 | 5 | 5 | 5 | 5 | 5 |
| Wasser | ad 100 | | | | | | | |

| ***Viskosität [mPas]*** | | | | | | | | |
|---|---|---|---|---|---|---|---|---|
| ***-sofort*** | 7.700 | 7.800 | 8.100 | 8.100 | 10.200 | 8.800 | 8.100 | 5.800 |
| ***-nach 7 d, 20°C*** | 7.600 | 7.800 | 5.900 | 5.000 | 6.000 | 6.300 | 7.100 | 4.500 |
| ***-nach 7 d, 40°C*** | 7.600 | 7.700 | 2.700 | 1.100 | 500 | 100 | 500 | 500 |

Man erkennt, daß lagerstabile Emulsionen nur unter Verwendung der erfindungsgemäßen ternären Kombinationen, d.h. unter Einsatz von Chitosan in Kombination mit Kokosmonoglyceridsulfat, erhalten werden.

## Patentansprüche

1. Kosmetische und/oder pharmazeutische Emulsionen, enthaltend - bezogen auf den Emulgatoranteil -
(a) 10 bis 50 Gew.-% Partialglycerid(ether)sulfate
(b) 50 bis 90 Gew.-% Emulgatoren und
(c) 0,1 bis 10 Gew.-% Chitosane,
mit der Maßgabe, daß sich die Mengenangaben zu 100 Gew.-% ergänzen.

2. Emulsionen nach Anspruch 1, **dadurch gekennzeichnet**, daß sie Partialglycerid(ether)sulfate der Formel **(I)** enthalten, 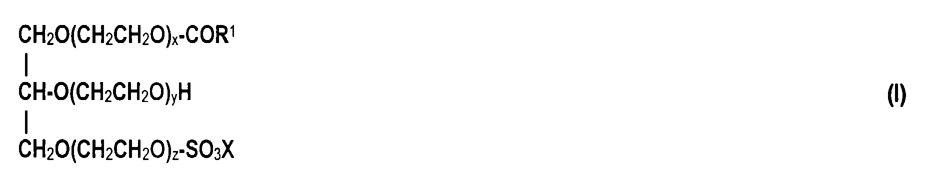 in der R¹CO für einen linearen oder verzweigten Acylrest mit 6 bis 22 Kohlenstoffatomen, x, y und z in Summe für 0 oder für Zahlen von 1 bis 30 und X für ein Alkali- oder Erdalkalimetall steht.

3. Emulsionen nach den Ansprüchen 1 und 2, **dadurch gekennzeichnet**, daß sie als Emulgatoren Alkyl- und/oder Alkenyloligoglykoside enthalten.

4. Emulsionen nach den Ansprüchen 1 bis 3, **dadurch gekennzeichnet**, daß sie Ölkörper enthalten, die ausgewählt sind aus der Gruppe, die gebildet wird von Guerbetalkoholen auf Basis von Fettalkoholen mit 6 bis 18 Kohlenstoffatomen, Estern von linearen C₆-C₂₀-Fettsäuren mit linearen C₆-C₂₀-Fettalkoholen, Estern von verzweigten C₆-C₁₃-Carbonsäuren mit linearen C₆-C₂₀-Fettalkoholen, Estern von linearen C₆-C₁₈-Fettsäuren mit verzweigten Alkoholen, Estern von linearen und/oder verzweigten Fettsäuren mit mehrwertigen Alkoholen und/oder Guerbetalkoholen, Triglyceriden auf Basis C₆-C₁₀-Fettsäuren, pflanzlichen Ölen, verzweigten primären Alkoholen, substituierten Cyclohexanen, Guerbetcarbonaten, Dialkylethern und/oder aliphatischen bzw. naphthenischen Kohlenwasserstoffen.

5. Emulsionen nach den Ansprüchen 1 bis 4, **dadurch gekennzeichnet**, daß sie einen nicht-wäßrigen Anteil von 5 bis 95 Gew.-% aufweisen.

6. Emulsionen nach den Ansprüchen 1 bis 5, **dadurch gekennzeichnet**, daß sie einen Emulgatoranteil von 0,1 bis 10 Gew.-% - bezogen auf den nicht-wäßrigen Anteil - aufweisen.
